# EUROPÄISCHE PATENTSCHRIFT

(11) **EP 3 937 427 B1**
(45) Veröffentlichungstag und Bekanntmachung des Hinweises auf die Patenterteilung: **21.02.2024**
(21) Anmeldenummer: 21183023.7
(22) Anmeldetag: 01.07.2021
(51) Int. Cl.: H04L 41/06, H04L 43/06

(54) **NETZWERKGERÄT UND MEDIZINSYSTEM ZUR DETEKTION MINDESTENS EINES NETZWERKPROBLEMS**
NETWORK DEVICE AND MEDICAL SYSTEM FOR DETECTING AT LEAST ONE NETWORK PROBLEM
APPAREIL DE RÉSEAU ET SYSTÈME MÉDICAL PERMETTANT DE DÉTECTER AU MOINS UN PROBLÈME DE RÉSEAU

(30) Priorität: 08.07.2020 DE 102020117984
(43) Veröffentlichungstag der Anmeldung: 12.01.2022
(73) Patentinhaber: Drägerwerk AG & Co. KGaA, 23558 Lübeck (DE)
(72) Erfinder: Marquardt, Klaus, 23558 Lübeck (DE); Molsen, Hannes, 23558 Lübeck (DE); Schurack, Harald, 23558 Lübeck (DE)

(56) Entgegenhaltungen:
- KR-A- 20150 007 966
- US-A1- 2014 077 956
- US-A1- 2020 134 165
- US-B1- 10 205 803

## Beschreibung

Die Erfindung betrifft ein Netzwerkgerät zur Detektion mindestens eines Netzwerkproblems in einem Medizinsystem. Weiterhin betrifft die Erfindung ein Medizinsystem mit mindestens einem Netzwerkgerät, ein Verfahren zur Detektion mindestens eines Netzwerkproblems in einem Medizinsystem und ein Computerprogramm mit einem Programmcode zur Durchführung eines solchen Verfahrens.

Es ist bekannt, dass ein Medizingerät eine entsprechende Warnmeldung ausgibt, wenn ein Netzwerkproblem durch das Medizingerät festgestellt wird. Weiterhin ist die Weiterleitung von einem Alarmsignal an Alarmgeber bekannt. So beschreibt DE 10 2015 009 087 A1 eine regionale und zeitliche Zuordnung von Alarmsignalen durch eine Alarmquelle zu einer Mehrzahl von Alarmgebern.

In US 2014 / 0 077 956 A1 ist ein Router für ein medizinisches Netzwerk offenbart, der dazu eingerichtet ist, einen Alarm abzugeben, wenn ein Netzwerkfehler vorliegt. Der Router ist dazu eingerichtet, die Verfügbarkeit von Netzverbindungskanälen zu überwachen, um das Vorliegen des Netzwerkfehlers zu erkennen.

In US 10 205 803 B1 ist ein Verfahren zur Bestimmung von Ursachen fehlgeschlagener Netzwerkverbindungen bekannt, wobei die Überwachung von TLS-Handshake-Fehlern vorgeschlagen wird.

Aufgabe der vorliegenden Erfindung ist es, ein verbessertes Netzwerkgerät, insbesondere ein Netzwerkgerät mit verbesserter Informationsweiterleitung bei der Detektion mindestens eines Netzwerkproblems, bereitzustellen.

Erfindungsgemäß wird zur Lösung dieser Aufgabe ein Netzwerkgerät zur Detektion mindestens eines Netzwerkproblems in einem Medizinsystem vorgeschlagen, mit einem Empfangsmodul, einem Überwachungsmodul und einem Sendemodul.

Das Empfangsmodul ist ausgebildet, aktuelle Prozessdaten mindestens eines innerhalb des Medizinsystems ausgeführten Prozesses zu empfangen.

Das Überwachungsmodul ist ausgebildet, basierend auf den Prozessdaten ein Vorliegen einer Anzahl von vorbestimmten Ereignissen zu detektieren, und es ist weiter ausgebildet, bei Vorliegen von mindestens einem der vorbestimmten Ereignisses eine Ausgabe eines entsprechenden Detektionssignals auszulösen.

Das Sendemodul ist ausgebildet, das Detektionssignal über ein mit dem Netzwerkgerät verbundenes Netzwerk an eine vorbestimmte Gerätadresse, insbesondere an ein vorbestimmtes Empfangsgerät, zu senden. Dabei umfasst die Anzahl von vorbestimmten Ereignissen mindestens eines der folgenden Ereignisse: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen; eine vorbestimmte Mehrzahl von Ausgaben über das Netzwerk ist innerhalb einer vorbestimmten zurückliegenden zweiten Zeitspanne ausgelöst; eine auszuführende Ausgabe eines Signals ist fehlgeschlagen; eine vorbestimmte Anzahl von Nachrichten im Rahmen einer Service-Discovery ist innerhalb einer vorbestimmten zurückliegenden dritten Zeitspanne empfangen.

Im Rahmen der Erfindung wurde erkannt, dass eine Alarmierung bei Vorliegen eines Netzwerkproblems an einem Medizingerät nicht den für die Reparatur dieses Netzwerkproblems relevanten Adressatenkreis erreicht oder diesen zumindest nur über Umwege erreicht. Daher wird erfindungsgemäß vorgeschlagen, bei Detektion eines Netzwerkproblems ein solches Detektionssignal an ein vorbestimmtes Empfangsgerät innerhalb des Netzwerks zu senden, welches sich vorzugsweise in räumlicher Nähe zu einer entsprechend geschulten Person aufhält.

Hierdurch wird der Betrieb des Medizinsystems vorteilhaft nicht durch die Feststellung des Netzwerkproblems unterbrochen oder gestört. Insbesondere während der Behandlung eines Patienten durch medizinisches Fachpersonal wird eine Ablenkung des Fachpersonals durch eine optische oder akustische Ausgabe der Meldung eines Netzwerkproblems vermieden.

Die vorbestimmten Ereignisse aus der Aufzählung von möglichen vorbestimmten Ereignissen indizieren verschiedene mögliche Netzwerkprobleme. Das Senden des Detektionssignals über das Sendemodul indiziert somit erfindungsgemäß das zumindest vermutete Vorliegen eines Netzwerkproblems.

Weiterhin erlaubt das erfindungsgemäße Netzwerkgerät eine sachgerechte Behebung des Netzwerkproblems. Insbesondere kann durch die automatisierte Weiterleitung des Detektionssignals an das Empfangsgerät, insbesondere an das mobile Empfangsgerät, eine zeitnahe Kontaktierung der für das Netzwerkproblems zuständigen Person erfolgen. Weiterhin kann durch die automatisierte Weiterleitung des Detektionssignals von der zuständigen Personen besonders gut eingeschätzt werden, wie schnell das Netzwerkproblem behoben werden muss. Hierdurch kann bereits kurz nach der Detektion des Netzwerkproblems der Ablauf einer möglichen Behebung dieses Problems, beispielsweise anhand eines intern vergebenen Dringlichkeitsstatus, festgelegt werden.

Die Prozessdaten sind erfindungsgemäß Daten, die Details zu dem innerhalb des Medizinsystems ausgeführten Prozess, insbesondere einzelne Prozessschritte indizieren, wie etwa Log-Einträge des Medizinsystems.

Module des erfindungsgemäßen Netzwerkgerätes können in einem gemeinsamen Gehäuse oder zumindest teilweise in separaten Gehäusen angeordnet sein. Alternativ oder ergänzend können zumindest Teile des Netzwerkgerätes in einem gemeinsamen Gehäuse mit Teilen des Medizinsystems angeordnet sein. Das Netzwerkgerät kann erfindungsgemäß separat von dem Medizinsystem ausgebildet sein und lediglich auf Daten des Medizinsystems, wie beispielsweise die Prozessdaten, zugreifen. Alternativ oder ergänzend kann das Netzwerkgerät ein Teil des Medizinsystems sein.

Die Module des Netzwerkgerätes können von einem gemeinsamen Prozessor ausgeführt werden, wobei sie erfindungsgemäß zumindest auf Software-Ebene voneinander getrennt sind.

Das Detektieren des Vorliegens von einem der vorbestimmten Ereignisse erfolgt erfindungsgemäß basierend auf den Prozessdaten. Hierbei kann das Detektieren beispielsweise auf einem Erkennen einer durch die Prozessdaten indizierten vorbestimmten Prozessschrittbeschreibung, einer durch die Prozessdaten indizierten vorbestimmten Prozesssignatur, eines vorbestimmten Begriffes innerhalb einer durch die Prozessdaten indizierten Prozessbeschreibung oder dergleichen basieren.

Vorzugsweise umfasst die Anzahl von vorbestimmten Ereignissen mindestens zwei der oben angegebenen Ereignisse. Die Anzahl von vorbestimmten Ereignissen kann erfindungsgemäß jede nicht-leere Untermenge der oben angegebenen Menge von Ereignissen umfassen.

Das Empfangsgerät ist erfindungsgemäß nicht Teil des Netzwerkgerätes. Es ist vorzugsweise räumlich getrennt von dem Netzwerkgerät angeordnet.

Bei der vorbestimmten Gerätadresse handelt es sich vorzugsweise um eine vorbestimmte Server-Adresse des für die entsprechende Benachrichtigung vorbestimmten Empfangsgeräts.

Das Netzwerk, mit dem das Netzwerkgerät kommuniziert, kann sowohl ein lokales Netzwerk, wie beispielsweise ein Krankenhaus-Netzwerk sein, als auch ein globales Netzwerk sein, dass eine Kommunikation mit Geräten außerhalb des Krankenhauses erlaubt. So kann das Empfangsgerät beispielsweise ein Empfangsgerät eines Servicetechnikers sein, der sich räumlich getrennt von dem Medizinsystem aufhält. Beispielsweise kann das Empfangsgerät ein mobiles Empfangsgerät, wie etwa ein Mobiltelefon, ein PC, ein Tablett, eine Smartwatch oder dergleichen sein.

Erfindungsgemäß umfassen die aktuellen Prozessdaten die entsprechenden Protokolldateien für den mindestens einen innerhalb des Medizinsystems ausgeführten Prozess. Protokolldateien sind in diesem Sinne beispielsweise Log-Einträge des Medizinsystems. Hierbei werden vorteilhaft Prozessdaten verwendet, die das Medizinsystem typischerweise sowieso ausgibt, um den Prozessablauf zu protokollieren. Hierdurch kann das erfindungsgemäße Netzwerkgerät besonders einfach in ein bestehendes Medizinsystem integriert werden. Nachfolgend werden bevorzugte Ausführungsformen des erfindungsgemäßen Netzwerkgeräts und weitere beispielhafte Merkmale beschrieben.

In einer besonders vorteilhaften Ausführungsform erfolgt das Senden des Detektionssignals an das vorbestimmte Empfangsgerät automatisiert innerhalb des genutzten Kommunikationsprotokolls, insbesondere über eine Simple Network Management Protocol-Trap (SNMP-Trap). Die Verwendung einer derartigen SNMP-Trap ist grundsätzlich bekannt für die Kommunikation innerhalb von Netzwerken. Daher wird auf die genaue Struktur eines derartigen Kommunikationsprotokolls im Folgenden nicht detailliert eingegangen.

In einer besonders bevorzugten Ausführungsform ist das Überwachungsmodul ausgebildet, basierend auf einer empfangenen Anzahl von Messwerten, insbesondere von physiologischen Messwerten, das Vorliegen mindestens eines physiologischen Ereignisses aus einer Anzahl von vorbestimmten physiologischen Ereignissen zu detektieren und abhängig von dieser Detektion ein entsprechendes Alarmierungssignal auszulösen. Vorzugsweise ist dabei das Sendemodul ausgebildet, das Alarmierungssignal über ein mit dem Netzwerkgerät verbundenes Netzwerk an mindestens ein vorbestimmtes Alarmierungsgerät zu senden. Dabei ist das vorbestimmte Empfangsgerät besonders bevorzugt verschieden von dem mindestens einen vorbestimmten Alarmierungsgerät. In diesem Ausführungsbeispiel wird besonders vorteilhaft unterschieden zwischen der Alarmierung über ein Alarmierungsgerät und einer Benachrichtigung über das Empfangsgerät für das Vorliegen eines vorbestimmten Ereignisses, das ein Netzwerkproblem anzeigt. In diesem Ausführungsbeispiel wird zwischen Alarmierungsgerät und Empfangsgerät unterschieden, sodass auch bezüglich der beiden Adressatenkreise zwischen der Patienten-indizierten Alarmierung und der Netzwerk-indizierten Benachrichtigung unterschieden wird.

Vorzugsweise ist das erfindungsgemäße Netzwerkgerät ein Medizingerät. Für ein solches Medizingerät ist besonders vorteilhaft, dass die Detektion eines Netzwerkproblems zur Kontaktierung eines separaten Empfangsgerätes führt, dass beispielsweise einem Systemadministrator, einem Servicemitarbeiter, oder einem anderen IT-Fachpersonal für das Medizingerät zugeordnet sein kann.

Vorzugsweise indiziert das Detektionssignal das detektierte Ereignis aus der Anzahl von vorbestimmten Ereignissen.

In einer Ausführungsform des erfindungsgemäßen Netzwerkgeräts wird die Anzahl von vorbestimmten Ereignissen von genau einem Ereignis gebildet. In dieser Ausführungsform zeigt das Detektionssignal bereits aufgrund des nur einen möglichen auslösenden Ereignisses das entsprechende Ereignis an. Vorteilhaft ist das Netzwerkgerät in dieser Ausführungsform zur Detektion eines einzigen konkreten Netzwerkproblems ausgebildet. Hierdurch ist die Auswertung des Detektionssignals besonders einfach.

Die vorbestimmte Mehrzahl von fehlgeschlagenen Passworteingabeversuchen umfasst vorzugsweise mindestens 2 Versuche, insbesondere mindestens 5 Versuche, besonders bevorzugt mehr als 5 Versuche. Die erste Zeitspanne umfasst weniger als 10 Minuten, insbesondere weniger als 5 Minuten, besonders bevorzugt höchstens 2 Minuten.

Die vorbestimmte ausgelöste Mehrzahl von Ausgaben über das Netzwerk umfasst vorzugsweise mindestens 50, insbesondere mindestens 75, besonders bevorzugt mindestens 100 Ausgaben. Die zweite Zeitspanne umfasst weniger als 5 Minuten, insbesondere weniger als 2 Minuten, besonders bevorzugt höchstens 30 Sekunden.

Die vorbestimmte Anzahl von Nachrichten im Rahmen einer Service-Discovery umfasst vorzugsweise mindestens 50 Nachrichten, insbesondere mindestens 75 Nachrichten, besonders bevorzugt mindestens 100 Nachrichten. Die dritte Zeitspanne umfasst vorzugsweise weniger als 30 Sekunden, insbesondere weniger als 10 Sekunden, besonders bevorzugt weniger als 6 Sekunden.

In einer vorteilhaften Ausführungsform ist das Netzwerkgerät ausgebildet, nach dem Senden eines ersten Detektionssignals für ein vorbestimmtes Zeitintervall kein zweites Detektionssignal zu senden, wenn das zweite Detektionssignal durch Detektion des gleichen vorbestimmten Ereignisses ausgelöst ist wie das erste Detektionssignal. In dieser Ausführungsform wird vermieden, dass der Adressatenkreis für die erfindungsgemäße Benachrichtigung, dass ein Netzwerkproblem vorliegt, mehrfach die gleiche Benachrichtigung für das gleiche Netzwerkproblem erhält. Hierdurch wird vermieden, dass ein Systemadministrator, ein Servicetechniker, eine IT-Fachperson oder dergleichen mehrfach aufgrund des gleichen Ereignisses abgelenkt wird. Erst nach Verstreichen des vorbestimmten Zeitintervalls wird der entsprechende Adressatenkreis erneut durch das Detektionssignal, also vorliegend durch das zweite Detektionssignal, auf das Vorliegen des entsprechenden Netzwerkproblems, also auf die Detektion eines vorbestimmten Ereignisses, hingewiesen. Hierdurch wird vorteilhaft sichergestellt, dass ein Empfangen des ersten Detektionssignals nicht vergessen wird. Ein zweites oder weiteres Detektionssignal wird vorzugsweise dann unabhängig von dem vorbestimmten Zeitintervall gesendet, wenn ein anderes vorbestimmtes Ereignis detektiert wurde, als das vorbestimmte Ereignis, dass zu dem ersten Detektionssignal geführt hat. Vorzugsweise ist das Überwachungsmodul dazu ausgebildet, das zweite Detektionssignal bei der Detektion des gleichen vorbestimmten Ereignisses entsprechend dieser Ausführungsform zu blockieren, bis das vorbestimmte Zeitintervall vergangen ist. In einer alternativen und/oder ergänzenden Variante dieser Ausführungsform ist das Sendemodul dazu ausgebildet, das zweite Detektionssignal bei der Detektion des gleichen vorbestimmten Ereignisses entsprechend dieser Ausführungsform zu blockieren, bis das vorbestimmte Zeitintervall vergangen ist. Das vorbestimmte Zeitintervall in dieser Ausführungsform beträgt vorzugsweise mindestens 10 Minuten, insbesondere mindestens 30 Minuten, besonders bevorzugt mindestens 2 Stunden.

Gemäß einem zweiten Aspekt der Erfindung wird zur Lösung des oben genannten Problems ein Medizinsystem mit mindestens einem Netzwerkgerät gemäß mindestens einem der vorhergehenden Ausführungsbeispiele und dem Empfangsgerät vorgeschlagen, wobei das Empfangsgerät über das Netzwerk mit dem Netzwerkgerät verbunden ist.

Das erfindungsgemäße Medizinsystem umfasst das erfindungsgemäße Netzwerkgerät und weist daher sämtliche Vorteile des erfindungsgemäßen Netzwerkgerätes auf.

Das Netzwerk ist beispielsweise ein Krankenhaus-Netzwerk, in dem eine Mehrzahl von Medizingeräten miteinander verbunden sind. Ein solches Krankenhaus-Netzwerk wird typischerweise von einem Systemadministrator oder einem anderen IT-Fachpersonal verwaltet. Vorzugsweise ist das Empfangsgerät einem solchen Systemadministrator oder einem solchen IT-Fachpersonal zugeordnet, damit die Benachrichtigung zum Anzeigen des Vorliegens eines Netzwerkproblems einen für die Behebung dieses Netzwerkproblems geeigneten Adressaten hat.

Vorzugsweise ist das Empfangsgerät ein mobiles Empfangsgerät. Hierdurch kann die für die Entgegennahme des Detektionssignals verantwortliche Person während ihrer gewöhnlichen Tätigkeit, die beispielsweise einen Ortswechsel beinhalten kann, über das Netzwerkproblem informiert werden. Dies ist insbesondere vorteilhaft, da ein solches Netzwerkproblem typischerweise ein seltenes Ereignis in solch einem Netzwerk ist.

In einer vorteilhaften Ausführungsform ist das vorbestimmte Empfangsgerät ein Verwaltungsmodul einer zentralen Netzwerkverwaltungseinheit des Medizinsystems. Ein derartiges Verwaltungsmodul ist vorteilhaft einer Fachperson für die Verwaltung einer solchen Netzwerkstruktur zugeordnet, so dass die erfindungsgemäße Benachrichtigung durch das Netzwerkgerät einen für die Behebung des Netzwerkproblems geeigneten Adressaten hat.

In einer besonders bevorzugten Ausführungsform weist das Medizinsystem das mindestens eine vorbestimmte Alarmierungsgerät auf. Hierdurch kann basierend auf Messwerten, wie beispielsweise physiologischen Messwerten, bei Vorliegen eines vorbestimmten physiologischen Ereignisses eine Alarmierung an dem Alarmierungsgerät ausgelöst werden. In einer Variante dieser Ausführungsform ist das mindestens eine vorbestimmte Alarmierungsgerät räumlich getrennt von dem vorbestimmten Empfangsgerät angeordnet. Hierdurch hat eine Alarmierung über das Alarmierungsgerät einen anderen Adressatenkreis als die erfindungsgemäße Benachrichtigung über das Empfangsgerät.

In einer besonders vorteilhaften Ausführungsform löst das Detektieren des Vorliegens mindestens eines vorbestimmten Ereignisses aus der Anzahl von vorbestimmten Ereignissen keine Ausgabe an einem zur Behandlung oder Untersuchung eines Patienten ausgebildeten Medizingerät aus. Hierdurch wird vermieden, dass medizinisches Fachpersonal beim Behandeln des Patienten abgelenkt wird durch eine akustische oder visuelle Ausgabe des Medizingerätes aufgrund des detektierten Netzwerkproblems. Typischerweise ist das medizinische Fachpersonal nicht dazu ausgebildet und/oder verfügt nicht über die notwendige Zeit, um ein solches Netzwerkproblem unter Verwendung des Medizingerätes zu lösen. Daher erlaubt diese Ausführungsform vorteilhaft eine Fokussierung des medizinischen Fachpersonals auf dessen medizinische Tätigkeit und eine gezielte Benachrichtigung des für das Netzwerk zuständigen Fachpersonals über das Empfangsgerät bei Vorliegen eines Netzwerkproblems.

Vorzugsweise ist das Empfangsgerät räumlich getrennt von durch das Medizinsystem zu behandelnden Patienten angeordnet. Hierdurch wird vorteilhaft weder der Patient gestört, wenn ein Netzwerkproblem detektiert wird, noch behandelndes medizinisches Fachpersonal gestört durch das Empfangsgerät. Vorzugsweise ist das Empfangsgerät dazu ausgebildet eine Ausgabe zu generieren, wenn es das erfindungsgemäße Detektionssignal empfängt. Eine solche Ausgabe ist in dieser Ausführungsform vorteilhaft nicht störend für einen in der Nähe des zu behandelnden Patienten befindlichen Personenkreis.

Gemäß einem dritten Aspekt der Erfindung wird zur Lösung des oben genannten Problems ein Verfahren zur Detektion mindestens eines Netzwerkproblems in einem Medizinsystem vorgeschlagen. Das erfindungsgemäß Verfahren weist die folgenden Schritte auf:
- Empfangen aktueller Prozessdaten mindestens eines innerhalb des Medizinsystems ausgeführten Prozesses;
- Detektieren eines Vorliegens einer Anzahl von vorbestimmten Ereignissen basierend auf den Prozessdaten;
- Auslösen eines entsprechenden Detektionssignals bei Vorliegen von mindestens einem der vorbestimmten Ereignisse;
- Senden des Detektionssignals über ein mit dem Netzwerkgerät verbundenes Netzwerk an eine vorbestimmte Gerätadresse, insbesondere an ein vorbestimmtes Empfangsgerät,
wobei die Anzahl von vorbestimmten Ereignissen mindestens eines der folgenden Ereignisse umfasst: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen; eine vorbestimmte Mehrzahl von Ausgaben über das Netzwerk ist innerhalb einer vorbestimmten zurückliegenden zweiten Zeitspanne ausgelöst; eine auszuführende Ausgabe eines Signals ist fehlgeschlagen; eine vorbestimmte Anzahl von Nachrichten im Rahmen einer Service-Discovery ist innerhalb einer vorbestimmten zurückliegenden dritten Zeitspanne empfangen, wobei die aktuellen Prozessdaten die entsprechenden Protokolldateien für den mindestens einen innerhalb des Medizinsystems ausgeführten Prozess umfassen.

Das erfindungsgemäß Verfahren wird durch das erfindungsgemäße Netzwerkgerät ausgeführt, sodass es sämtliche Vorteile des Netzwerkgerätes gemäß dem ersten Aspekt der Erfindung aufweist.

In einer besonders bevorzugten Ausführungsform weist das erfindungsgemäße Verfahren weiterhin die Schritte auf:
- Detektieren eines physiologischen Ereignisses aus einer Anzahl von vorbestimmten physiologischen Ereignissen basierend auf einer empfangenen Anzahl von Messwerten, insbesondere von physiologischen Messwerten;
- Auslösen eines entsprechenden Alarmierungssignals abhängig von dieser Detektion;
- Senden des Alarmierungssignals über ein mit dem Netzwerkgerät verbundenes Netzwerk an mindestens ein vorbestimmtes Alarmierungsgerät, wobei das vorbestimmte Empfangsgerät verschieden von dem mindestens einen vorbestimmten Alarmierungsgerät, insbesondere räumlich getrennt von dem mindestens einen vorbestimmten Alarmierungsgerät, ist.

In dieser Ausführungsform erlaubt das erfindungsgemäße Verfahren das Vorsehen von zwei verschiedenen Adressatenkreisen, nämlich einem Adressatenkreis mit Alarmierungsgerät, welches basierend auf physiologischen Ereignissen alarmiert wird, und einem Adressatenkreis mit Empfangsgerät, welches basierend auf Prozessdaten, die ein mögliches Netzwerkproblem indizieren, benachrichtigt wird. Der zu alarmierende Adressatenkreis umfasst vorzugsweise ein medizinisches Fachpersonal, wohingegen der zu benachrichtigen Adressatenkreis einen Systemadministrator, Service-Personal, IT-Fachpersonal oder dergleichen umfasst.

Gemäß einem vierten Aspekt der Erfindung wird zur Lösung der oben genannten Aufgabe ein Computerprogramm mit einem Programmcode zur Durchführung eines erfindungsgemäßen Verfahrens gemäß einer der vorhergehenden Ausführungsformen vorgeschlagen, wenn der Programmcode auf einem Computer, einem Prozessor oder einer programmierbaren Hardwarekomponente ausgeführt wird. Vorzugsweise werden mehrere Schritte des erfindungsgemäßen Verfahrens durch einen gemeinsamen Computer, einen gemeinsamen Prozessor oder eine gemeinsame programmierbare Hardwarekomponente ausgeführt. Vorzugsweise sind die einzelnen Schritte dabei zumindest auf Software-Ebene voneinander durch entsprechende Softwareblöcke getrennt. Besonders bevorzugt werden alle Schritte des erfindungsgemäßen Verfahrens auf einem gemeinsamen Computer, einem gemeinsamen Prozessor oder einer gemeinsamen programmierbaren Hardwarekomponente ausgeführt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels eines Netzwerkgerätes gemäß einem ersten Aspekt der Erfindung;
- Fig. 2: eine schematische Darstellung eines zweiten Ausführungsbeispiels des Netzwerksgerätes gemäß dem ersten Aspekt der Erfindung;
- Fig. 3: eine schematische Darstellung eines Ausführungsbeispiels eines Medizinsystems gemäß einem zweiten Aspekt der Erfindung;
- Fig. 4: ein Flussdiagramm eines Ausführungsbeispiels eines Verfahrens gemäß einem dritten Aspekt der Erfindung.

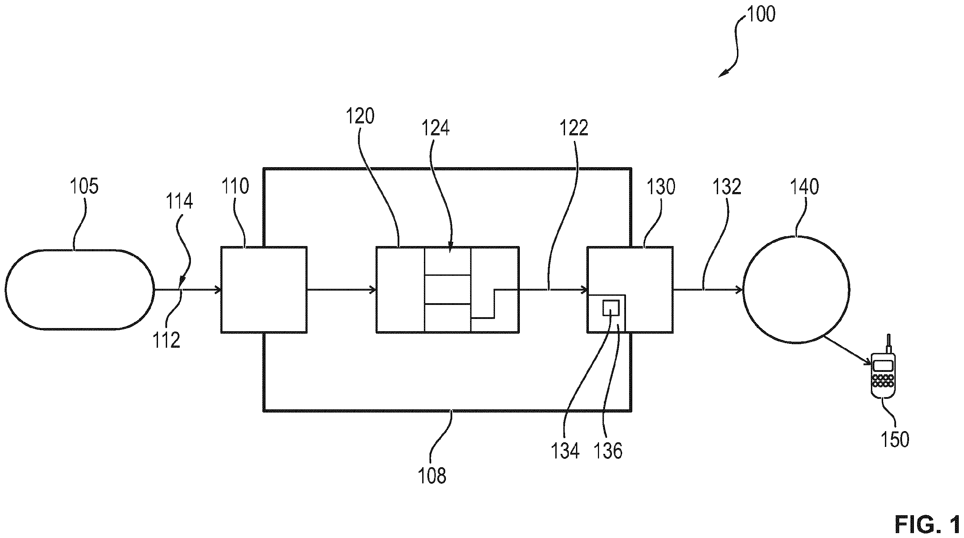

Fig. 1 zeigt eine schematische Darstellung eines ersten Ausführungsbeispiels eines Netzwerkgerätes 100 gemäß einem ersten Aspekt der Erfindung.

Das Netzwerkgerät 100 ist zur Detektion mindestens eines Netzwerkproblems in einem Medizinsystem 105 ausgebildet. Hierfür umfasst es ein Empfangsmodul 110, ein Überwachungsmodul 120 und ein Sendemodul 130. Das Netzwerkgerät 100 ist in dem dargestellten Ausführungsbeispiel kein Teil des Medizinsystems 105. Die Kommunikation zwischen dem Medizinsystem 105 und dem Netzwerkgerät 100 erfolgt durch einen Kommunikationskanal mit dem Empfangsmodul 110. Dieser Kommunikationskanal kann ein Teil des Netzwerks 140 sein, mit dem das Netzwerkgerät 100 verbunden ist. Alternativ oder ergänzend kann der Kommunikationskanal mit dem Medizinsystem 105 ein direkter kabelbasierte oder kabellose Kommunikationskanal sein.

Das Empfangsmodul 110 ist ausgebildet, aktuelle Prozessdaten 112 mindestens eines innerhalb des Medizinsystems 105 ausgeführten Prozesses zu empfangen. In dem dargestellten Ausführungsbeispiel handelt es sich bei den Prozessdaten 112 um Log-Einträge eines Medizingerätes, dass innerhalb des Medizinsystems 105 ausgeführt wird. Das Empfangsmodul 110 wandelt die aktuellen Prozessdaten 112, die es durch ein Datensignal 114 von dem Medizinsystem 105 empfängt, in für das Überwachungsmodul 120 verarbeitbare Daten um und leitet diese zu dem Überwachungsmodul 120 weiter. Eine derartige Umwandlung kann auch ein identisches Weiterleiten der Prozessdaten umfassen.

Das Überwachungsmodul 120 ist ausgebildet, basierend auf den Prozessdaten 112 ein Vorliegen einer Anzahl von vorbestimmten Ereignissen 124 zu detektieren. Insbesondere ist das Überwachungsmodul 120 ausgebildet, die Prozessdaten 112 auf eine vorbestimmte Weise zu analysieren, um das Vorliegen eines Ereignisses aus einer vorbestimmten Gruppe von Ereignissen zu detektieren. Ein solches kann über eine Auswertung eines Wortes, einer Signatur, einer Zeichenfolge oder dergleichen innerhalb der Prozessdaten 112 erfolgen. Schließlich ist das Überwachungsmodul 120 weiter ausgebildet, beim Vorliegen mindestens eines solchen vorbestimmten Ereignisses 124 eine Ausgabe eines entsprechenden Detektionssignals 132 auszulösen. Das Auslösen des Detektionssignals 132 erfolgt über ein Auslösesignal 122, das an das Sendemodul 130 ausgegeben wird.

Die von dem Überwachungsmodul 120 überwachten vorbestimmten Ereignisse 124 umfassen mindestens eines der folgenden Ereignisse: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen; eine vorbestimmte Mehrzahl von Ausgaben über das Netzwerk ist innerhalb einer vorbestimmten zurückliegenden zweiten Zeitspanne ausgelöst; eine auszuführende Ausgabe eines Signals ist fehlgeschlagen; eine vorbestimmte Anzahl von Nachrichten im Rahmen einer Service-Discovery ist innerhalb einer vorbestimmten zurückliegenden dritten Zeitspanne empfangen.

In dem dargestellten Ausführungsbeispiel werden die folgenden drei vorbestimmten Ereignisse 124 überwacht: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen; eine vorbestimmte Anzahl von Nachrichten im Rahmen einer Service-Discovery ist innerhalb einer vorbestimmten zurückliegenden dritten Zeitspanne empfangen.

Bei der fehlgeschlagenen Verschlüsselung handelt es sich insbesondere um einen fehlgeschlagenen Transport Layer Security Handshake (TLS Handshake), der ein zuverlässiges Anzeichen für ein vorliegendes Netzwerkproblem bildet.

Die vorbestimmte zurückliegenden ersten Zeitspanne beträgt vorzugsweise weniger als 10 Minuten, insbesondere weniger als 5 Minuten, besonders bevorzugt höchstens 2 Minuten. Die vorbestimmte Mehrzahl von Passworteingabeversuchen beträgt vorzugsweise mindestens drei, insbesondere mindestens fünf, besonders bevorzugt mehr als fünf.

Das Sendemodul 130 ist ausgebildet, das Detektionssignal 132 über das mit dem Netzwerkgerät verbundene Netzwerk 140 an eine vorbestimmte Gerätadresse 134, insbesondere an ein vorbestimmtes Empfangsgerät 150, zu senden. Die Gerätadresse ist dabei vorliegend auf einem Speicher 136 des Sendemoduls 130 hinterlegt. In einem alternativen oder ergänzenden Ausführungsbeispiel ist die vorbestimmte Gerätadresse außerhalb des Sendemoduls und innerhalb des Netzwerkgerätes hinterlegt, wie beispielsweise innerhalb des Überwachungsmoduls.

Bei dem Empfangsgerät 150 handelt es sich um das der Gerätadresse 134 zugeordnete Endgerät. Die vorbestimmte Gerätadresse 134 ist beispielsweise eine Server-Adresse, eine IP-Adresse oder dergleichen von dem Empfangsgerät 150. Das Empfangsgerät 150 ist vorliegend ein mobiles Endgerät, wie etwa ein Mobiltelefon oder dergleichen. Das Senden des Detektionssignals 132 erfolgt automatisiert innerhalb des genutzten Kommunikationsprotokolls, insbesondere über eine Simple Network Management Protocol-Trap (SNMP-Trap). Beispiele für eine mögliche Umsetzung einer solchen SNMP-Trap sind dem Fachmann bekannt und werden daher im Folgenden nicht erläutert.

Vorliegend weist das Netzwerkgerät 100 zudem ein Gehäuse 108 auf, welches die Module des Netzwerkgerätes 100 vor äußeren Einwirkungen geschützt.

Bei dem Netzwerk 140 handelt es sich vorliegend um ein Krankenhausnetzwerk. Über das Krankenhausnetzwerk sind verschiedene Medizingeräte miteinander vernetzt. Mindestens eines dieser Medizingeräte ist in dem Medizinsystem 105 enthalten, dass die Prozessdaten 112 für das erfindungsgemäße Netzwerkgerät 100 bereitstellt.

Die Module des Netzwerkgerätes 100 sind vorzugsweise kabelbasiert miteinander verbunden. Dabei können die Module von einem gemeinsamen Prozessor des Netzwerkgerätes 100 ausgeführt werden, wobei sie zumindest auf Software-Ebene voneinander getrennt sind.

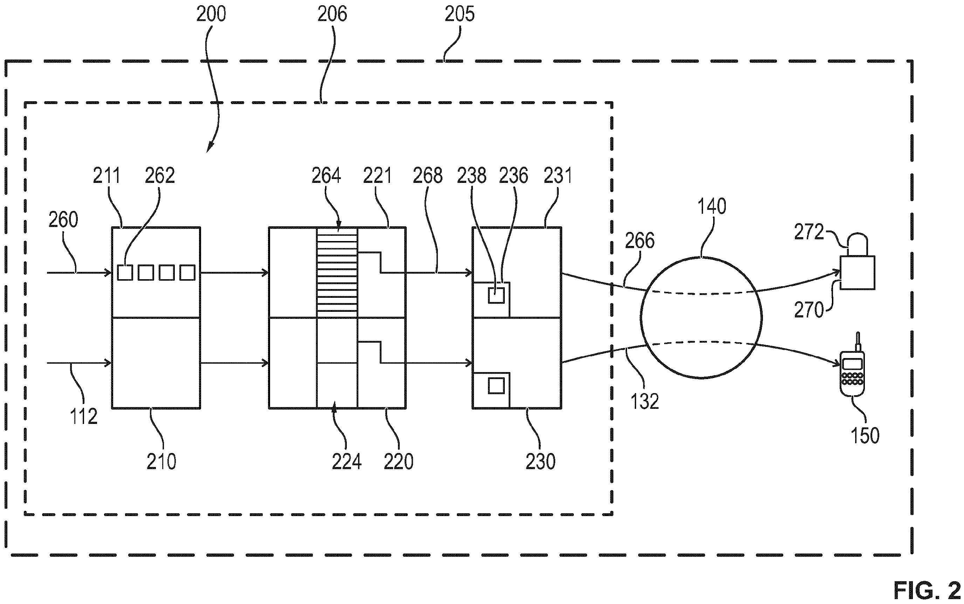

Fig. 2 zeigt eine schematische Darstellung eines zweiten Ausführungsbeispiels des Netzwerksgerätes 200 gemäß dem ersten Aspekt der Erfindung.

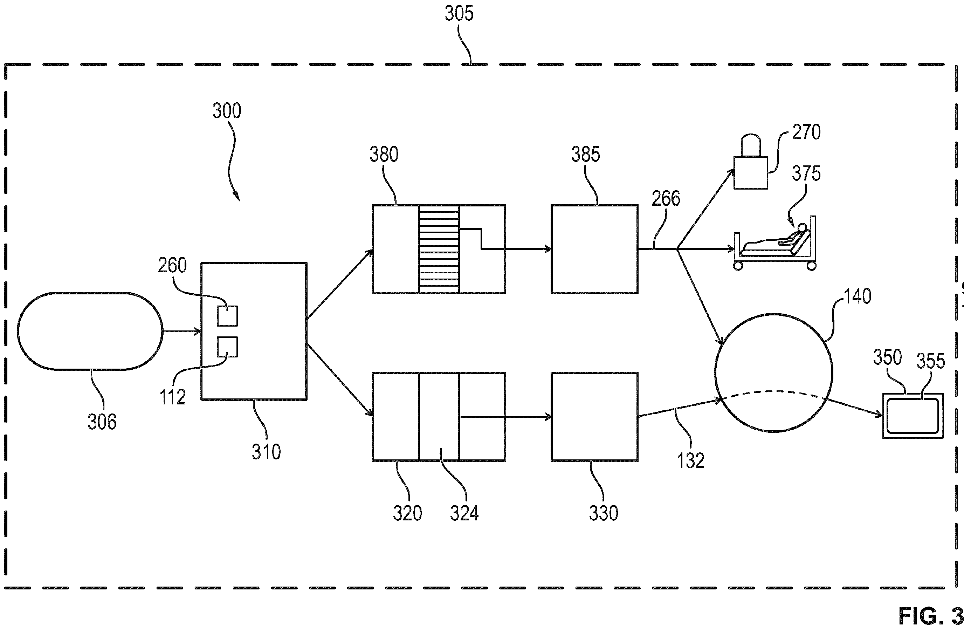

Das Netzwerkgerät 200 unterscheidet sich dadurch von dem in Fig. 1 dargestellten Netzwerkgerät 100, das es einen Teil des Medizinsystems 205 bildet. Die Prozessdaten 112 werden mithin innerhalb des Medizinsystems 205 versendet, um das Empfangsmodul 210 zu erreichen. Das Empfangsmodul 210 weist einen weiteren Empfangsbereich 211 auf, die dazu ausgebildet ist Messwertdaten 260 zu empfangen, die eine Anzahl von Messwerten 262, insbesondere von physiologischen Messwerten, indizieren. In dem dargestellten Ausführungsbeispiel werden die Prozessdaten 112 über eine andere Schnittstelle durch das Empfangsmodul 210 empfangen als die Messwertedaten 260. In einem alternativen oder ergänzenden Ausführungsbeispiel, wie beispielsweise dem in Fig. 3 dargestellten Ausführungsbeispiel, können diese Daten über eine gemeinsame Schnittstelle empfangen werden.

Das Überwachungsmodul 220 weist entsprechend einen weiteren Überwachungsbereichs 221 auf, der ausgebildet ist das Vorliegen mindestens eines physiologischen Ereignisses aus einer Anzahl von vorbestimmten physiologischen Ereignissen 264 zu detektieren. Die Anzahl von vorbestimmten physiologischen Ereignissen 264 ist größer als die Anzahl von vorbestimmten Ereignissen 224, die ein Netzwerkproblem indizieren. Dies liegt darin begründet, dass bei der Behandlung eines Patienten viele verschiedene den Patienten betreffende Ereignisse eintreten können, die durch Messwerte, insbesondere durch physiologische Messwerte indiziert werden.

Falls ein solches physiologisches Ereignis detektiert wird, ist das Überwachungsmodul 220 mit den weiteren Überwachungsbereichs 221 dazu ausgebildet, ein Alarmierungssignal 266 auszulösen, welches durch das Sendemodul 230 ausgegeben wird. Das Auslösen des Alarmierungssignals 266 erfolgt durch ein weiteres Auslösesignal 268.

Das Sendemodul 230 weist entsprechend einen weiteren Sendebereich 231 auf, der ausgebildet ist, das Alarmierungssignal 266 nach dem Empfangen des Ausgabesignals 268 zu senden, wobei dieses Alarmierungssignal 266 über das mit dem Netzwerkgerät verbundenen Netzwerk 140 an mindestens eine vorbestimmtes Alarmierungsadresse 238 gesendet wird, die ein entsprechendes Alarmierungsgerät 270 indiziert. Dabei ist das Alarmierungsgerät 270 verschieden von dem vorbestimmten Empfangsgerät 150. Die Alarmierungsadresse 238 ist in einem Speicher 236 des Sendemodul 230 hinterlegt.

Das Empfangsgerät 150 und das Alarmierungsgerät 270 sind ebenfalls ein Teil des Medizinsystems 205. Dabei sind das Empfangsgerät 150 und das Alarmierungsgerät 270 räumlich getrennt voneinander angeordnet. Vorzugsweise steht das Empfangsgerät 150 an einem Ort, der für die Verwaltung der IT-Infrastruktur einer das Medizinsystem aufweisenden Einrichtung, wie etwa eines Krankenhauses oder eines Pflegeheims, vorgesehen ist. Vorzugsweise ist das Empfangsgerät 150 räumlich getrennt von einer durch das Medizinsystem 205 zu behandelnden Person angeordnet. Typischerweise befindet sich der Adressat für das ein Netzwerkproblem anzeigende Detektionssignal, nämlich ein Systemadministrator, ein Servicemitarbeiter, eine IT-Fachkraft oder dergleichen, nicht in einer Umgebung des zu behandelnden Patienten. Daher braucht eine durch das Detektionssignal 132 ausgelöste Ausgabe nicht in der Umgebung des zu behandelnden Patienten zu erfolgen. Das Alarmierungsgerät 270 befindet sich hingegen vorzugsweise in der Nähe eines medizinischen Fachpersonals, wie etwa in einem Zimmer für Krankenpflegepersonal. In dem dargestellten Ausführungsbeispiel ist das Alarmierungsgerät 270 ein Gerät mit einer Leuchte 272, die eine optische Ausgabe nach dem Empfang des Alarmierungssignals 266 über das Netzwerk 140 bereitstellt.

In dem dargestellten Ausführungsbeispiel wird das Detektionssignal 132 ausschließlich an das Empfangsgerät 150 gesendet. In einem nicht dargestellten Ausführungsbeispiel wird das Detektionssignal an eine Gruppe von Empfangsgeräten gesendet, wie etwa an eine Gruppe von Empfangsgeräten die einer Gruppe von Servicetechniker zugeordnet sind.

In dem dargestellten Ausführungsbeispiel wird das Alarmierungssignal 266 ausschließlich an das Alarmierungsgerät 270 ausgegeben. In einem nicht dargestellten Ausführungsbeispiel wird das Alarmierungssignal an eine Gruppe von Alarmierungsgeräten gesendet, wie etwa an eine Gruppe von mobilen Geräten, die jeweils einem medizinischen Fachpersonal zugeordnet sind.

In dem dargestellten Ausführungsbeispiel ist das Netzwerkgerät 200 ein Teil eines Medizingerätes 206, wie etwa eines Beatmungsgerätes, eines Patienten-Monitors, einer Patienten-Überwachungseinrichtung oder dergleichen.

Die von dem Überwachungsmodul 220 überwachten vorbestimmten Ereignisse 224 umfassen mindestens eines der folgenden beiden Ereignisse: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen.

Das Überwachungsmodul 220 ist in dem dargestellten Ausführungsbeispiel weiter ausgebildet, nach dem Senden des ersten Detektionssignals 132 für ein vorbestimmtes Zeitintervall kein zweites Detektionssignal zu senden, wenn das zweite Detektionssignal durch Detektion des gleichen vorbestimmten Ereignisses 224 ausgelöst ist wie das erste Detektionssignal 132. Das vorbestimmte Zeitintervalle beträgt hierbei mindestens 10 Minuten, vorzugsweise mindestens 30 Minuten, besonders bevorzugt mindestens 2 Stunden. Hierdurch wird vermieden, dass ein Netzwerkproblem zu einer andauernden, kurzfristig wiederkehrenden Benachrichtigung des Empfangsgeräts 150 führt. Unter dem ersten Detektionssignal 132 ist im Sinne der Erfindung ein Detektionssignals verstehen, das zeitlich vor dem zweiten Detektionssignal zu senden ist.

Fig. 3 zeigt eine schematische Darstellung eines Ausführungsbeispiels eines Medizinsystems 305 gemäß einem zweiten Aspekt der Erfindung.

Das Medizinsystem 305 unterscheidet sich dadurch von dem Medizinsystem 205 aus Fig. 2, dass das Empfangsmodul 310 Daten von einem Medizingerät 306 empfängt, die sowohl Prozessdaten 112 als auch Messwertdaten 260 umfassen. Das Empfangsmodul 310 ist dazu ausgebildet, aus den empfangenen Daten die Prozessdaten 112 zu bestimmen und an das Überwachungsmodul 320 weiterzuleiten, und zudem aus den empfangenen Daten die Messwertdaten 260 zu bestimmen und an ein separates Alarmierungsüberwachungsmodul 380 zu senden. Das Alarmierungsüberwachungsmodul 380 arbeitet genauso wie der weitere Überwachungsbereich 221 des Überwachungsmoduls 220 aus Fig. 2. das Überwachungsmodul 320 unterscheidet sich von den bisher beschriebenen Überwachungsmodulen 120, 220 dadurch, dass das Überwachungsmodul 320 nur das Auftreten eines einzigen vorbestimmten Ereignisses 324 überwacht, nämlich das Ereignis, das eine vorbestimmte Mehrzahl von Passworteingabeversuchen innerhalb einer vorbestimmten zurückliegenden ersten Zeitspanne fehlgeschlagen ist. In einem alternativen nicht dargestellten Ausführungsbeispiel wird lediglich das Ereignis überwacht, dass eine Verschlüsselung im Rahmen eines in dem Netzwerk verwendeten Verschlüsselungsprotokolls fehlgeschlagen ist. In wiederum einem weiteren alternativen nicht dargestellten Ausführungsbeispiel wird lediglich das Ereignis überwacht, dass eine vorbestimmte Mehrzahl von Ausgaben über das Netzwerk innerhalb einer vorbestimmten zurückliegenden zweiten Zeitspanne ausgelöst ist. In wiederum einem weiteren alternativen nicht dargestellten Ausführungsbeispiel wird lediglich das Ereignis überwacht, dass eine vorbestimmte Anzahl von Nachrichten im Rahmen einer Service-Discovery innerhalb einer vorbestimmten zurückliegenden dritten Zeitspanne empfangen wurde. Die vorbestimmte Anzahl von Nachrichten umfasst vorzugsweise mehr als 50, insbesondere mehr als 75, besonders bevorzugt mehr als 100 Nachrichten. Die dritte Zeitspanne ist vorzugsweise kürzer als 30 Sekunden, insbesondere kürzer als 10 Sekunden, vorzugsweise weniger als 6 Sekunden.

Weiterhin unterscheidet sich das Medizinsystem 305 mit dem erfindungsgemäßen Netzwerkgerät 300 dadurch, dass ein Alarmierungssendemodul 385 separat zu dem Sendemodul 330 ausgebildet ist. Das Sendemodul 330 ist entsprechend dem Sendemodul 130 aus Fig. 1 ausgebildet, wobei das Empfangsgerät 350 in diesem Ausführungsbeispiel ein Verwaltungsmodul einer zentralen Netzwerkverwaltungseinheit des Medizinsystems 305 ist. Das Verwaltungsmodul weist dabei ein Display 355 als Ausgabemedium auf.

Das Alarmierungssendemodul 385 ist dazu ausgebildet, direkt an das Alarmierungsgerät 270 das entsprechende Alarmierungssignal 266 zu senden. Weiterhin wird das Alarmierungssignal 266 an eine Ausgabeeinheit in der Nähe des Patienten 375 gesendet und an das Netzwerk 140 ausgegeben. Die Ausgabe an das Netzwerk 140 stellt sicher, dass eventuell innerhalb des entsprechenden Krankenhauses genutzte Alarmierungssysteme auf das Alarmierungssignal 266 über das Netzwerk zugreifen können. Für die Benachrichtigung des Empfangsgerätes 350 durch das Detektionssignal 132 ist eine derartige allgemeine Hinterlegung in dem Netzwerk 140 nicht vorgesehen, sondern lediglich eine gezielte Benachrichtigung des Empfangsgerätes 350, um gezielt die vorbestimmte Adressatengruppe über das wahrscheinlich vorliegenden Netzwerkproblem zu informieren.

Vorzugsweise wird das Detektionssignal 132 nicht an ein zur Behandlung oder Untersuchung des Patienten 375 ausgebildetes Medizingerät gesendet oder löst an diesem Medizingerät zumindest keine Ausgabe aus. Hierdurch wird vermieden, dass medizinisches Fachpersonal durch eine Information abgelenkt wird, die bei einem anderen Adressatenkreis, wie etwa dem Systemadministrator, zu einer schnelleren Behebung des Netzwerkproblems führen kann.

Das Alarmierungsüberwachungsmodul 380 und das Alarmierungssendemodul 385 sind in dem dargestellten Ausführungsbeispiel von dem Netzwerkgerät räumlich getrennte Module. In einem nicht dargestellten Ausführungsbeispiel sind die beiden Module räumlich getrennt von dem entsprechenden Überwachungsmodul und dem entsprechenden Sendemodul des Netzwerkgerätes, bilden aber dennoch ein Bestandteil des erfindungsgemäßen Netzwerkgerätes. In einem weiteren nicht dargestellten Ausführungsbeispiel sind die beiden Module entsprechende Bestandteile des Überwachungsmoduls und des Sendemoduls des erfindungsgemäßen Netzwerkgerätes.

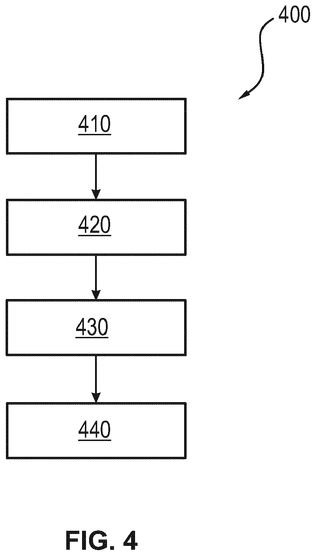

Fig. 4 zeigt ein Flussdiagramm eines Ausführungsbeispiels eines Verfahrens 400 gemäß einem dritten Aspekt der Erfindung.

Das erfindungsgemäße Verfahren 400 ist zur Detektion mindestens eines Netzwerkproblems in einem Medizinsystem ausgebildet. Hierfür weist es die im Folgenden angegebenen Verfahrensschritte auf.

Ein erster Schritt 410 umfasst ein Empfangen aktueller Prozessdaten mindestens eines innerhalb des Medizinsystems ausgeführten Prozesses.

Ein nächster Schritt 420 umfasst ein Detektieren eines Vorliegens einer Anzahl von vorbestimmten Ereignissen basierend auf den Prozessdaten. Hierbei umfasst die Anzahl von vorbestimmten Ereignissen erfindungsgemäß mindestens eines der folgenden Ereignisse: eine vorbestimmte Mehrzahl von Passworteingabeversuchen ist innerhalb einer vorbestimmten zurückliegenden ersten Zeitspanne fehlgeschlagen; eine Verschlüsselung im Rahmen eines in dem Netzwerk verwendeten Verschlüsselungsprotokolls ist fehlgeschlagen; eine vorbestimmte Mehrzahl von Ausgaben über das Netzwerk ist innerhalb einer vorbestimmten zurückliegenden zweiten Zeitspanne ausgelöst; eine auszuführende Ausgabe eines Signals ist fehlgeschlagen; eine vorbestimmte Anzahl von Nachrichten im Rahmen einer Service-Discovery ist innerhalb einer vorbestimmten zurückliegenden dritten Zeitspanne empfangen.

Ein darauffolgender Schritt 430 umfasst ein Auslösen eines entsprechenden Detektionssignals bei Vorliegen mindestens eines vorbestimmten Ereignisses.

Ein abschließender Schritt 440 umfasst ein Senden des Detektionssignals über ein mit dem Netzwerkgerät verbundenes Netzwerk an eine vorbestimmte Gerätadresse, insbesondere an ein vorbestimmtes Empfangsgerät.

Die Schritte 410, 420, 430 und 440 werden vorzugsweise in dieser Reihenfolge ausgeführt. Der Schritt 410 führt nur dann zu den weiteren Schritten des Verfahrens 400, wenn ein vorbestimmtes Ereignis basierend auf den im Schritt 410 empfangenen Prozessdaten detektiert wird. Im Rahmen einer solchen Detektion wird stets der Schritt 420 ausgeführt und nach einer solchen Detektion werden stets die Schritte 430 und 440 in dieser Reihenfolge ausgeführt. Nach dem Auslösen des Detektionssignals im Schritt 430 erfolgt unmittelbar das Senden des Detektionssignals an die vorbestimmte Gerätadresse im Schritt 440.

Da die vorliegend genannten möglichen vorbestimmten Ereignisse ein Netzwerkproblem anzeigen, treten sie typischerweise selten auf, sodass auf den Schritt 410 nur selten der Schritt 420, nämlich das Detektieren eines vorbestimmten Ereignisses folgt.

Nach dem Senden des Detektionssignals entsprechend Schritt 440, wird dieser Schritt 440 vorzugsweise für ein vorbestimmtes Zeitintervall nicht mehr ausgeführt, falls das letzte Detektionssignal durch eine Detektion des gleichen vorbestimmten Ereignisses ausgelöst wurde, wie das aktuell zu sendende Detektionssignal. Das vorbestimmte Zeitintervall beträgt vorzugsweise mindestens 10 Minuten, besonders bevorzugt mindestens 30 Minuten, insbesondere mindestens 2 Stunden.

In einem besonders bevorzugten Ausführungsbeispiel werden die Schritte des Verfahrens 400 ergänzt um die folgenden Verfahrensschritte:
- Detektieren eines physiologischen Ereignisses aus einer Anzahl von vorbestimmten physiologischen Ereignissen basierend auf einer empfangenen Anzahl von Messwerten, insbesondere von physiologischen Messwerten;
- Auslösen eines entsprechenden Alarmierungssignals abhängig von dieser Detektion;
- Senden des Alarmierungssignals über ein mit dem Netzwerkgerät verbundenes Netzwerk an mindestens ein vorbestimmtes Alarmierungsgerät, wobei das vorbestimmte Empfangsgerät verschieden von dem mindestens einen vorbestimmten Alarmierungsgerät, insbesondere räumlich getrennt von dem mindestens einen vorbestimmten Alarmierungsgerät, ist.

In diesem bevorzugten Ausführungsbeispiel wird zwischen zwei verschiedenen Adressatenkreisen für verschiedene Ausgaben unterschieden. Eine Alarmierung über das Alarmierungssignal erreicht einen ersten Adressatenkreis, der sich im Bereich des Alarmierungsgerätes aufhält. Eine Benachrichtigung über das Detektionssignal erreicht hingegen eines zweiten Adressatenkreis der sich im Bereich des Empfangsgerätes, welches verschieden von dem Alarmierungsgerät ist, aufhält. Der erste Adressatenkreis umfasst vorzugsweise medizinisches Fachpersonal, wohingegen der zweite Adressatenkreis vorzugsweise einen Systemadministrator, Service-Personal, eine IT-Fachkraft oder dergleichen umfasst. In diesem bevorzugten Ausführungsbeispiel wird daher sichergestellt, dass das Netzwerkproblem nicht das medizinische Fachpersonal ablenkt, welches zum Beheben des Netzwerkproblems typischerweise nicht ausgebildet ist, sondern dass das Netzwerkproblem schnell und zuverlässig einen zur Behebung dieses Netzwerkproblems geeigneten Adressaten erreicht.

### Bezugszeichenliste

- 100, 200, 300: Netzwerkgerät
- 105, 205, 305: Medizingerät
- 206, 306: Medizingerät
- 108: Gehäuse
- 110, 210, 310: Empfangsmodul
- 112: Prozessdaten
- 114: Datensignal
- 120, 220, 320: Überwachungsmodul
- 122: Auslösesignal
- 124, 224, 324: vorbestimmte Ereignisse
- 130, 230, 330: Sendemodul
- 132: Detektionssignal
- 134: Gerätadresse
- 136, 236: Speicher
- 140: Netzwerk
- 150, 350: Empfangsgerät
- 211: weiterer Empfangsbereich
- 221: weiterer Überwachungsbereich
- 231: weiterer Sendebereich
- 238: Alarmierungsadresse
- 260: Messwertdaten
- 262: Messwert
- 264: physiologische Ereignisse
- 266: Alarmierungssignal
- 268: weiteres Auslösesignal
- 270: Alarmierungsgerät
- 272: Leuchte
- 355: Display
- 375: Patient
- 380: Alarmierungsüberwachungsmodul
- 385: Alarm ierungssendemodul
- 400: Verfahren
- 410, 420, 430, 440: Verfahrensschritte

## Patentansprüche

1. Netzwerkgerät (100) zur Detektion mindestens eines Netzwerkproblems in einem Medizinsystem (105), mit
- einem Empfangsmodul (110), das ausgebildet ist, aktuelle Prozessdaten (112) mindestens eines innerhalb des Medizinsystems (105) ausgeführten Prozesses zu empfangen,
- einem Überwachungsmodul (120), das ausgebildet ist, basierend auf den Prozessdaten (112) ein Vorliegen einer Anzahl von vorbestimmten Ereignissen (124) zu detektieren, und das weiter ausgebildet ist, bei Vorliegen von mindestens einem der vorbestimmten Ereignisse (124) eine Ausgabe eines entsprechenden Detektionssignals (132) auszulösen, und
- einem Sendemodul (130), das ausgebildet ist, das Detektionssignal (132) über ein mit dem Netzwerkgerät (100) verbundenes Netzwerk (140) an eine vorbestimmte Gerätadresse (134), insbesondere an ein vorbestimmtes Empfangsgerät (150), zu senden,
wobei die Anzahl von vorbestimmten Ereignissen (124) mindestens eines der folgenden Ereignisse umfasst:
eine vorbestimmte Mehrzahl von Passworteingabeversuchen ist innerhalb einer vorbestimmten zurückliegenden ersten Zeitspanne fehlgeschlagen;
eine Verschlüsselung im Rahmen eines in dem Netzwerk (140) verwendeten Verschlüsselungsprotokolls ist fehlgeschlagen;
eine vorbestimmte Mehrzahl von Ausgaben über das Netzwerk (140) ist innerhalb einer vorbestimmten zurückliegenden zweiten Zeitspanne ausgelöst;
eine auszuführende Ausgabe eines Signals ist fehlgeschlagen;
eine vorbestimmte Anzahl von Nachrichten im Rahmen einer Service-Discovery ist innerhalb einer vorbestimmten zurückliegenden dritten Zeitspanne empfangen, und
wobei die aktuellen Prozessdaten (112) die entsprechenden Protokolldateien für den mindestens einen innerhalb des Medizinsystems (105) ausgeführten Prozess umfassen.

2. Netzwerkgerät (100) gemäß Anspruch 1, wobei das Senden des Detektionssignals (132) an das vorbestimmte Empfangsgerät (150) automatisiert innerhalb eines genutzten Kommunikationsprotokolls, insbesondere über eine Simple Network Management Protocol-Trap (SNMP-Trap), erfolgt.

3. Netzwerkgerät (200) gemäß mindestens einem der vorhergehenden Ansprüche, wobei das Überwachungsmodul (220) ausgebildet ist, basierend auf einer empfangenen Anzahl von Messwerten (262), insbesondere von physiologischen Messwerten, das Vorliegen mindestens eines physiologischen Ereignisses aus einer Anzahl von vorbestimmten physiologischen Ereignissen (264) zu detektieren und abhängig von dieser Detektion ein entsprechendes Alarmierungssignal (266) auszulösen, und wobei das Sendemodul (230) ausgebildet ist, das Alarmierungssignal (266) über ein mit dem Netzwerkgerät (200) verbundenes Netzwerk (140) an mindestens ein vorbestimmtes Alarmierungsgerät (270) zu senden, und wobei das vorbestimmte Empfangsgerät (150) verschieden von dem mindestens einen vorbestimmten Alarmierungsgerät (270) ist.

4. Netzwerkgerät (200) gemäß mindestens einem der vorhergehenden Ansprüche, wobei das Netzwerkgerät (200) ausgebildet ist, nach dem Senden eines ersten Detektionssignals (132) für ein vorbestimmtes Zeitintervall kein zweites Detektionssignal zu senden, wenn das zweite Detektionssignal durch Detektion des gleichen vorbestimmten Ereignisses (124) ausgelöst ist wie das erste Detektionssignal (132).

5. Medizinsystem (205) mit
- mindestens einem Netzwerkgerät (200) gemäß mindestens einem der vorhergehenden Ansprüche und
- dem Empfangsgerät (150), das über das Netzwerk (140) mit dem Netzwerkgerät (200) verbunden ist.

6. Medizinsystem (305) gemäß Anspruch 5, wobei das vorbestimmte Empfangsgerät (350) ein Verwaltungsmodul einer zentralen Netzwerkverwaltungseinheit des Medizinsystems (305) ist.

7. Medizinsystem (205) gemäß Anspruch 5 oder 6 mit einem Netzwerkgerät (200) gemäß Anspruch 4, weiterhin aufweisend das mindestens eine vorbestimmte Alarmierungsgerät (270), wobei das mindestens eine vorbestimmte Alarmierungsgerät (270) räumlich getrennt von dem vorbestimmten Empfangsgerät (150) angeordnet ist.

8. Medizinsystem (305) gemäß mindestens einem der Ansprüche 5 bis 7, wobei das Detektieren des Vorliegens mindestens eines vorbestimmten Ereignisses (124) aus der Anzahl von vorbestimmten Ereignissen (124) keine Ausgabe an einem zur Behandlung oder Untersuchung eines Patienten (375) ausgebildeten Medizingerät auslöst.

9. Medizinsystem (305) gemäß mindestens einem der Ansprüche 5 bis 8, wobei das Empfangsgerät (350) räumlich getrennt von durch das Medizinsystem (305) zu behandelnden Patienten (375) angeordnet ist.

10. Verfahren (400) zur Detektion mindestens eines Netzwerkproblems in einem Medizinsystem (105), aufweisend die Schritte
- Empfangen aktueller Prozessdaten (112) mindestens eines innerhalb des Medizinsystems (105) ausgeführten Prozesses;
- Detektieren eines Vorliegens einer Anzahl von vorbestimmten Ereignissen (124) basierend auf den Prozessdaten (112);
- Auslösen eines entsprechenden Detektionssignals (132) bei Vorliegen von mindestens einem der vorbestimmten Ereignisse (124);
- Senden des Detektionssignals (132) über ein mit dem Netzwerkgerät (100) verbundenes Netzwerk (140) an eine vorbestimmte Gerätadresse (134), insbesondere an ein vorbestimmtes Empfangsgerät (150),
wobei die Anzahl von vorbestimmten Ereignissen (124) mindestens eines der folgenden Ereignisse umfasst:
eine vorbestimmte Mehrzahl von Passworteingabeversuchen ist innerhalb einer vorbestimmten zurückliegenden ersten Zeitspanne fehlgeschlagen;
eine Verschlüsselung im Rahmen eines in dem Netzwerk (140) verwendeten Verschlüsselungsprotokolls ist fehlgeschlagen;
eine vorbestimmte Mehrzahl von Ausgaben über das Netzwerk (140) ist innerhalb einer vorbestimmten zurückliegenden zweiten Zeitspanne ausgelöst;
eine auszuführende Ausgabe eines Signals ist fehlgeschlagen;
eine vorbestimmte Anzahl von Nachrichten im Rahmen einer Service-Discovery ist innerhalb einer vorbestimmten zurückliegenden dritten Zeitspanne empfangen,
wobei die aktuellen Prozessdaten (112) die entsprechenden Protokolldateien für den mindestens einen innerhalb des Medizinsystems (105) ausgeführten Prozess umfassen.

11. Verfahren gemäß Anspruch 10, weiterhin aufweisend die Schritte
- Detektieren eines physiologischen Ereignisses (264) aus einer Anzahl von vorbestimmten physiologischen Ereignissen (264) basierend auf einer empfangenen Anzahl von Messwerten (262), insbesondere von physiologischen Messwerten;
- Auslösen eines entsprechenden Alarmierungssignals (266) abhängig von dieser Detektion;
- Senden des Alarmierungssignals (266) über ein mit dem Netzwerkgerät (100) verbundenes Netzwerk (140) an mindestens ein vorbestimmtes Alarmierungsgerät (270), wobei das vorbestimmte Empfangsgerät (150) verschieden von dem mindestens einen vorbestimmten Alarmierungsgerät (270), insbesondere räumlich getrennt von dem mindestens einen vorbestimmten Alarmierungsgerät (270), ist.

12. Computerprogramm mit einem Programmcode zur Durchführung eines Verfahrens (400) gemäß Anspruch 10 oder 11, wenn der Programmcode auf einem Computer, einem Prozessor oder einer programmierbaren Hardwarekomponente ausgeführt wird.

## Claims

1. Network device (100) for detecting at least one network problem in a medical system (105), the device having
- a receiving module (110) which is designed to receive current process data (112) of at least one process which is performed within the medical system (105),
- a monitoring module (120) which is designed to detect the presence of a number of predetermined events (124) on the basis of the process data (112) and is further designed to trigger an output of a corresponding detection signal (132) when at least one of the predetermined events (124) is present, and
- a transmitting module (130) which is designed to transmit the detection signal (132) to a predetermined device address (134), in particular to a predetermined receiving device (150), via a network (140) which is connected to the network device (100),
wherein the number of predetermined events (124) comprises at least one of the following events:
a predetermined plurality of password input attempts is unsuccessful within a predetermined past first time period;
encryption within the context of an encryption protocol used in the network (140) is unsuccessful;
a predetermined plurality of outputs via the network (140) is triggered within a predetermined past second time period;
an output of a signal to be carried out is unsuccessful;
a predetermined number of messages within the context of a service discovery is received within a predetermined past third time period, and
wherein the current process data (112) comprise the corresponding protocol files for the at least one process which is performed within the medical system (105).

2. Network device (100) according to claim 1, wherein the detection signal (132) is transmitted to the predetermined receiving device (150) automatically within a used communication protocol, in particular via a simple network management protocol trap (SNMP trap).

3. Network device (200) according to at least one of the preceding claims, wherein the monitoring module (220) is designed to detect the presence of at least one physiological event from a number of predetermined physiological events (264) on the basis of a received number of measured values (262), in particular physiological measured values, and to trigger a corresponding alarm signal (266) depending on this detection, and wherein the transmitting module (230) is designed to transmit the alarm signal (266) to at least one predetermined alarm device (270) via a network (140) which is connected to the network device (200), and wherein the predetermined receiving device (150) is different from the at least one predetermined alarm device (270).

4. Network device (200) according to at least one of the preceding claims, wherein the network device (200) is designed not to transmit a second detection signal for a predetermined time interval after transmitting a first detection signal (132) if the second detection signal is triggered by detecting the same predetermined event (124) as the first detection signal (132).

5. Medical system (205) having
- at least one network device (200) according to at least one of the preceding claims, and
- the receiving device (150) which is connected to the network device (200) via the network (140).

6. Medical system (305) according to claim 5, wherein the predetermined receiving device (350) is a management module of a central network management unit of the medical system (305).

7. Medical system (205) according to either claim 5 or claim 6, having a network device (200) according to claim 4 and further comprising the at least one predetermined alarm device (270), wherein the at least one predetermined alarm device (270) is arranged so as to be spatially separated from the predetermined receiving device (150).

8. Medical system (305) according to at least one of claims 5 to 7, wherein detecting the presence of at least one predetermined event (124) from the number of predetermined events (124) does not trigger an output at a medical device which is designed for treating or examining a patient (375).

9. Medical system (305) according to at least one of claims 5 to 8, wherein the receiving device (350) is arranged so as to be spatially separated from patients (375) to be treated by the medical system (305).

10. Method (400) for detecting at least one network problem in a medical system (105), comprising the steps of
- receiving current process data (112) of at least one process which is performed within the medical system (105);
- detecting a presence of a number of predetermined events (124) on the basis of the process data (112);
- triggering a corresponding detection signal (132) when at least one of the predetermined events (124) is present;
- transmitting the detection signal (132) to a predetermined device address (134), in particular to a predetermined receiving device (150), via a network (140) which is connected to the network device (100),
wherein the number of predetermined events (124) comprises at least one of the following events:
a predetermined plurality of password input attempts is unsuccessful within a predetermined past first time period;
encryption within the context of an encryption protocol used in the network (140) is unsuccessful;
a predetermined plurality of outputs via the network (140) is triggered within a predetermined past second time period;
an output of a signal to be carried out is unsuccessful;
a predetermined number of messages within the context of a service discovery is received within a predetermined past third time period,
wherein the current process data (112) comprise the corresponding protocol files for the at least one process which is performed within the medical system (105).

11. Method according to claim 10, further comprising the steps of
- detecting a physiological event (264) from a number of predetermined physiological events (264) on the basis of a received number of measured values (262), in particular physiological measured values;
- triggering a corresponding alarm signal (266) depending on this detection;
- transmitting the alarm signal (266) to at least one predetermined alarm device (270) via a network (140) which is connected to the network device (100), wherein the predetermined receiving device (150) is different from the at least one predetermined alarm device (270), in particular is spatially separated from the at least one predetermined alarm device (270).

12. Computer program having a program code for carrying out a method (400) according to either claim 10 or claim 11 when the program code is executed on a computer, a processor, or a programmable hardware component.

## Revendications

1. Appareil réseau (100) permettant la détection d'au moins un problème de réseau dans un système médical (105), comportant
- un module de réception (110) qui est configuré pour recevoir des données de processus (112) actuelles d'au moins un processus exécuté dans le système médical (105),
- un module de surveillance (120) qui est configuré pour détecter, sur la base des données de processus (112), la présence d'un certain nombre d'événements prédéterminés (124) et qui est en outre configuré pour déclencher, en cas de présence d'au moins l'un des événements prédéterminés (124), une émission d'un signal de détection (132) correspondant, et
- un module d'envoi (130) qui est configuré pour envoyer le signal de détection (132) à une adresse d'appareil (134) prédéterminée, en particulier à un appareil de réception (150) prédéterminé, par l'intermédiaire d'un réseau (140) connecté à l'appareil réseau (100),
dans lequel le nombre d'événements prédéterminés (124) comprend au moins l'un des événements suivants :
une pluralité prédéterminée de tentatives de saisie de mot de passe a échoué dans une première période de temps prédéterminée passée ;
un chiffrement dans le cadre d'un protocole de chiffrement utilisé dans le réseau (140) a échoué ;
une pluralité prédéterminée d'émissions sur le réseau (140) est déclenchée dans une deuxième période de temps prédéterminée passée ;
une émission à exécuter d'un signal a échoué ;
un nombre prédéterminé de messages dans le cadre d'une découverte de services est reçu dans une troisième période de temps prédéterminée passée, et
dans lequel les données de processus (112) actuelles comprennent les fichiers journaux correspondants pour l'au moins un processus exécuté dans le système médical (105).

2. Appareil réseau (100) selon la revendication 1, dans lequel l'envoi du signal de détection (132) à l'appareil de réception (150) prédéterminé est effectué de manière automatisée dans un protocole de communication utilisé, en particulier par l'intermédiaire d'un trap de protocole simple de gestion de réseau (trap SNMP).

3. Appareil réseau (200) selon au moins l'une des revendications précédentes, dans lequel le module de surveillance (220) est configuré pour détecter, sur la base d'un nombre reçu de valeurs de mesure (262), en particulier de valeurs de mesure physiologiques, la présence d'au moins un événement physiologique parmi un nombre d'événements physiologiques (264) prédéterminés, et pour déclencher un signal d'alarme (266) correspondant en fonction de ladite détection, et dans lequel le module d'envoi (230) est configuré pour envoyer le signal d'alarme (266) à au moins un appareil d'alarme (270) prédéterminé par l'intermédiaire d'un réseau (140) connecté à l'appareil réseau (200), et dans lequel l'appareil de réception (150) prédéterminé est différent de l'au moins un appareil d'alarme (270) prédéterminé.

4. Appareil réseau (200) selon au moins l'une des revendications précédentes, dans lequel l'appareil réseau (200) est configuré pour ne pas envoyer un second signal de détection après l'envoi d'un premier signal de détection (132) pendant un intervalle de temps prédéterminé si le second signal de détection est déclenché par la détection du même événement prédéterminé (124) que celui du premier signal de détection (132).

5. Système médical (205) comportant
- au moins un appareil réseau (200) selon au moins l'une des revendications précédentes et
- l'appareil de réception (150) qui est connecté à l'appareil réseau (200) par l'intermédiaire du réseau (140).

6. Système médical (305) selon la revendication 5, dans lequel l'appareil de réception (350) prédéterminé est un module de gestion d'une unité centrale de gestion de réseau du système médical (305).

7. Système médical (205) selon la revendication 5 ou 6, comportant un appareil réseau (200) selon la revendication 4, présentant en outre l'au moins un appareil d'alarme (270) prédéterminé, dans lequel l'au moins un appareil d'alarme (270) prédéterminé est disposé de manière à être séparé spatialement de l'appareil de réception (150) prédéterminé.

8. Système médical (305) selon au moins l'une des revendications 5 à 7, dans lequel la détection de la présence d'au moins un événement prédéterminé (124) parmi le nombre d'événements prédéterminés (124) ne déclenche pas d'émission sur un appareil médical configuré pour traiter ou examiner un patient (375).

9. Système médical (305) selon au moins l'une des revendications 5 à 8, dans lequel l'appareil de réception (350) est disposé de manière à être séparé spatialement de patients (375) à traiter par le système médical (305).

10. Procédé (400) permettant la détection d'au moins un problème de réseau dans un système médical (105), présentant les étapes consistant à
- recevoir des données de processus (112) actuelles d'au moins un processus exécuté dans le système médical (105) ;
- détecter la présence d'un certain nombre d'événements prédéterminés (124) sur la base des données de processus (112) ;
- déclencher un signal de détection (132) correspondant en présence d'au moins l'un des événements prédéterminés (124) ;
- envoyer le signal de détection (132) à une adresse d'appareil (134) prédéterminée, en particulier à un appareil de réception (150) prédéterminé, par l'intermédiaire d'un réseau (140) connecté à l'appareil réseau (100),
dans lequel le nombre d'événements prédéterminés (124) comprend au moins l'un des événements suivants :
une pluralité prédéterminée de tentatives de saisie de mot de passe a échoué dans une première période de temps prédéterminée passée ;
un chiffrement dans le cadre d'un protocole de chiffrement utilisé dans le réseau (140) a échoué ;
une pluralité prédéterminée d'émissions sur le réseau (140) est déclenchée dans une deuxième période de temps prédéterminée passée ;
une émission à exécuter d'un signal a échoué ;
un nombre prédéterminé de messages dans le cadre d'une découverte de services est reçu dans une troisième période de temps prédéterminée passée,
dans lequel les données de processus (112) actuelles comprennent les fichiers journaux correspondants pour l'au moins un processus exécuté dans le système médical (105).

11. Procédé selon la revendication 10, présentant en outre les étapes consistant à
- détecter un événement physiologique (264) parmi un nombre d'événements physiologiques (264) prédéterminés sur la base d'un nombre reçu de valeurs de mesure (262), en particulier de valeurs de mesure physiologiques ;
- déclencher un signal d'alarme (266) correspondant en fonction de ladite détection ;
- envoyer le signal d'alarme (266) à au moins un appareil d'alarme (270) prédéterminé par l'intermédiaire d'un réseau (140) connecté à l'appareil réseau (100), dans lequel l'appareil de réception (150) prédéterminé est différent de l'au moins un appareil d'alarme (270) prédéterminé, en particulier est séparé spatialement de l'au moins un appareil d'alarme (270) prédéterminé.

12. Programme informatique comportant un code de programme pour la mise en oeuvre d'un procédé (400) selon la revendication 10 ou 11, lorsque le code de programme est exécuté sur un ordinateur, un processeur ou un composant matériel programmable.
